# EUROPEAN PATENT SPECIFICATION

(11) **EP 1 193 272 B1**
(45) Date of publication and mention of the grant of the patent: **30.06.2004**
(21) Application number: 01121651.2
(22) Date of filing: 13.09.2001
(51) Int. Cl.: C07K 14/62, C12N 15/17, C12N 5/10, A61K 38/28, A61K 48/00

(54) **Single-chain insulin analogs**
Einkettige Insulinanaloge
Analogues d'insuline à base d'une chaine simple

(30) Priority: 02.10.2000 KR 2000058003; 07.11.2000 US 706690
(43) Date of publication of application: 03.04.2002
(73) Proprietor: Yonsei University, 120-749 Seoul (KR)
(72) Inventor: Lee, Hyun Chul, Dongdo-academyhouse A-402, Seoul (KR); Kim, Su-Jin, Haibit 1819-1304, Goyangsi (KR); Kim, Kyung-Sup, Seoul (KR); Shin, Hang-Cheol, Seoul (KR); Yoon, Ji-Won, Calgary, Alberta T3A 4X5 (CA)
(74) Representative: Reinhard - Skuhra - Weise & Partner

(56) References cited:
- EP-A- 0 741 188
- US-A- 5 962 267
- CHANG S-G ET AL: "Human insulin production from a novel mini-proinsulin which has high receptor-binding activity" BIOCHEMICAL JOURNAL, PORTLAND PRESS, LONDON, GB, vol. 329, 1998, pages 631-635, XP001037629 ISSN: 0264-6021
- LEE ET AL: "Remission in models of type 1 diabetes by gene therapy using a single-chain insulin analogue" NATURE,MACMILLAN MAGAZINES LTD, vol. 408, 23 November 2000 (2000-11-23), pages 483-488, XP002186755

## Description

### BACKGROUND OF THE INVENTION

The present invention relates to a method of introducing at least one single-chain insulin analog protein or a gene encoding a single-chain insulin analog (SIA) into at least one mammalian tissue for use in treating diabetes in the mammalian host. The present invention also relates to the single-chain insulin analog and a recombinant vector construct comprising the gene encoding SIA.

The cure of diabetes has long been sought using several different approaches, including islet transplantation, regeneration of β cells and insulin gene therapy (Levine, F. & Leibowitz, G. Towards gene therapy of diabetes mellitus. *Mol*. *Med*. *Today* 5, 165-171 (1999)). However, the permanent remission of type 1 diabetes has not yet been satisfactorily achieved.

WO96/34882 discusses making single chain insulin with high bioactivity, but does not disclose the single chain insulin analog of the invention.

Single chain insulin analogs with high bioactivity were also reported, but the analogs can be refolded only at non-physiological pH, above pH 9.5 (US-A-5962267; Biochemical Journal, 1998, 631-635). EP-A-741188 describes secretion of a single chain insulin analog in the pancreatic beta cell line in response to physiological levels of glucose, but the activity of the secreted single chain insulin analog or in vivo animal experiment was not disclosed.

There remains a very real and substantial need for a method of introducing at least one gene encoding a single-chain insulin analog to at least one cell of a mammalian host *in vitro* or *in vivo*, for use in treating the mammalian host suffering from diabetes.

### SUMMARY OF THE INVENTION

The present invention has met the hereinbefore described need.

A method of introducing at least one gene encoding a product into at least one cell of a mammalian tissue for use in treating a mammalian host is provided in the present invention.

This method includes employing recombinant techniques to produce a DNA vector molecule containing the gene coding for the product and introducing the DNA vector molecule containing the gene coding for the product into the tissue cell. The DNA vector molecule can be any DNA molecule capable of being delivered and maintained within the target cell or tissue such that the gene encoding the product of interest can be stably expressed. The DNA vector molecule preferably utilized in the present invention is either a viral or plasmid DNA vector molecule. This method preferably includes introducing the gene encoding the product into the cell of the mammalian tissue for a therapeutic use.

An object of the invention is to provide a single-chain insulin analog compound of formula (I) having the properties of greater insulin receptor binding activity than proinsulin and less insulin receptor binding activity than insulin:

B chain - Uₗ-Zₙ-Yₘ-Zₗ-Uₙ - A chain (I)

wherein:
B and A chains are the human insulin chains, respectively, and
U is an arginine or lysine residue;
Z is glycine;
Y is a peptide as defined herein below;
I is an integer of 2-n; and
n is an integer of 0 or 2;

In this compound, Y is glycine-proline-glycine, or alanine-proline-glycine-aspartic acid-valine, or tyrosine-proline-glycine-aspartic acid-valine, or histidine-proline-glycine-aspartic acid-valine.

Another object of the invention is to provide a polynucleotide encoding the single-chain insulin analog described above. Another embodiment of the invention includes a recombinant vector comprising the polynucleotide that encodes the single chain insulin analog described above. The vector may be a plasmid or a virus. If a virus, preferably, it is adeno-associated virus. Moreover, it is preferred that the promoter be inducible. More preferably, the promoter may be regulated by glucose. Even more preferably, the promoter is a pyruvate kinase gene promoter. Most preferably, the promoter is the hepatocyte-specific L-type pyruvate kinase gene promoter.

The invention is also directed to a cell line transformed with the above-described vector.

Another embodiment of the invention is directed to a method for treating a patient suffering from diabetes comprising:
a) generating a recombinant viral or plasmid vector comprising a polynucleotide encoding a single-chain insulin analog operatively linked to a promoter; and
b) introducing said recombinant viral or plasmid vector to said patient, such that expression of said polynucleotide within said patient results in remission of diabetes.

Preferably, the viral vector is adeno-associated virus, and the promoter is an inducible promoter. Preferably, the promoter is regulated by glucose. In the method described above, preferably the dosage of said viral vector is at least about 10¹¹ viral particles. Preferably, the treatment method is accomplished by using a vector that is introduced to the patient through the cell line comprising the single chain insulin analog described above.

The present invention is also directed to a method for treating a patient suffering from diabetes comprising administering the single chain insulin analog compound described above to a patient in need thereof. Preferably, the diabetes is type I diabetes.

These and other objects of the invention will be more fully understood from the following description of the invention, the referenced drawings attached hereto and the claims appended hereto.

### BRIEF DESCRIPTION OF THE DRAWINGS

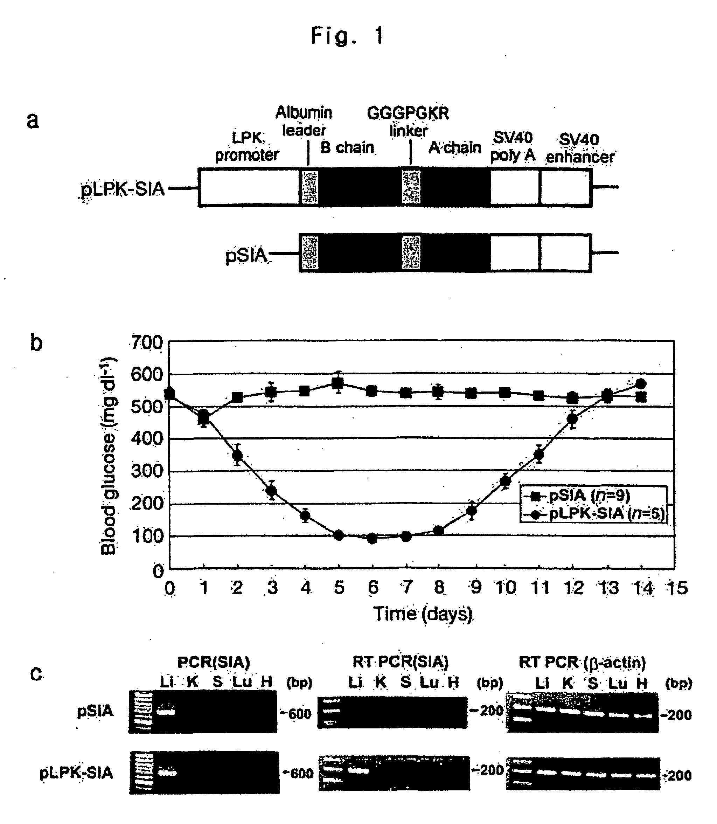

Figures 1A-1C. - Construction of pLPK-SIA, hypoglycemic effect of pLPK-SIA and hepatocyte-specific expression of pLPK-SIA.
1A. Diagram of pLPK-SIA construct showing LPK promoter, albumin leader sequence, SIA cDNA, SV40 poly(A) sequence and SV40 enhancer and diagram of pSIA.
1B. Transient hypoglycemic effect of pLPK-SIA. When blood glucose levels in STZ-induced diabetic rats were over 500 mg/dl, 30 µg of pSIA or pLPK-SIA was mixed with 60 µl of FuGENE™ (a 6 cationic multimeric fusion gene from Boehringer Mannheim, Mannheim, Germany) and injected into the hepatic portal vein, and blood glucose levels were measured. Each time point represents the mean ± SD value.
1C. SIA DNA is detected in both pSIA- and pLPK-SIA-treated rats, but SIA is expressed only in pLPK-SIA-treated rats. DNA or total RNA for PCR or RT-PCR was isolated from the liver (Li), kidney (K), spleen (S), lung (L) and heart (H) of rats 5 days after treatment with pSIA or pLPK-SIA.

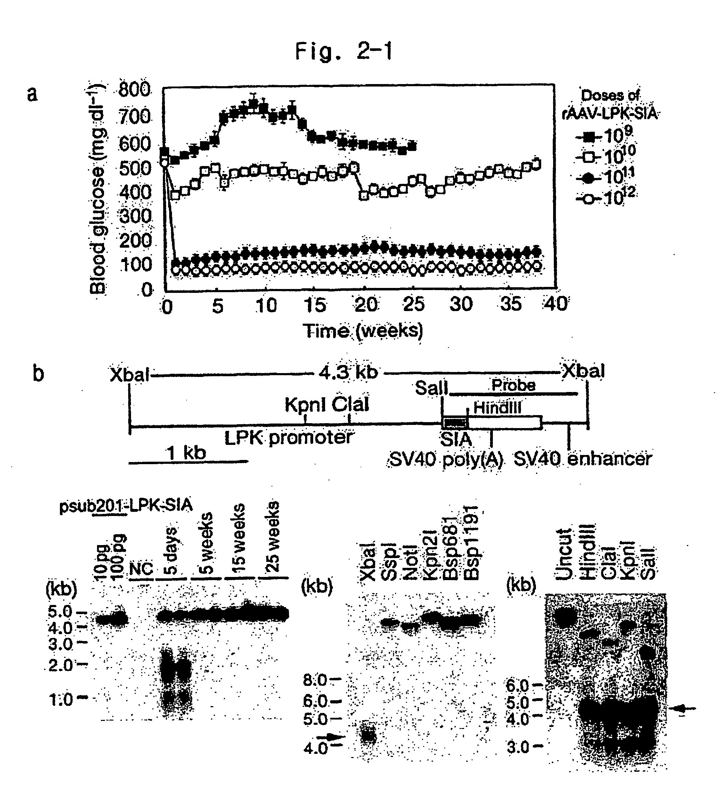

Figures 2A-2J. - Hypoglycemic effect of rAAV-LPK-SIA, integration of rAAV-LPK-SIA into chromosomal DNA in hepatocytes and expression of SIA.
2A. Different doses of rAAV-LPK-SIA (1 x 10⁹ - 1 x 10¹² particles) were injected into the hepatic portal vein of STZ-induced diabetic SD rats (11-13 weeks old, > 500 mg/dl blood glucose; n = 10/group), and blood glucose levels were measured up to 38 weeks after rAAV-LPK-SIA treatment. Each time point represents the mean ± SD value. The mean blood glucose levels of normal control SD rats (n = 10) was 103 ± 10 mg/dl. All animals treated with 10⁹ particles died at 25 weeks after treatment, whereas 30% of the animals treated with 10¹⁰ particles remained alive at 38 weeks after treatment.
2B. The restriction enzyme map of LPK-SIA and the region that was used as a probe are shown. Total cellular DNA was isolated from the livers of STZ-induced diabetic SD rats at 5 days, 5 weeks, 15 weeks and 25 weeks after administration of 1 x 10¹¹ virus particles of rAAV-LPK-SIA. After digestion of chromosomal DNA with XbaI, Southern blot hybridization was performed using a ³²P-labelled probe containing the SIA, SV40 poly(A) and SV40 enhancer sequences. The XbaI-digested psub201-LPK-SIA (10 pg, 100 pg) and chromosomal DNA from normal control SD rats (NC) were used as a positive and negative control, respectively.

The digestion of DNA from hepatocytes at 25 weeks after rAAV-LPK-SIA treatment with non-cutting enzymes that have no restriction sites in the rAAV-LPK-SIA genome showed differently sized, single molecular weight bands that were much larger than the 4.3 Kb band (arrow), suggesting that rAAV-LPK-SIA DNA is integrated into chromosomal DNA (center panel). The digestion with single cutting enzymes showed a 4.3 Kb band (arrow) and one differently sized band, suggesting that the rAAV-LPK-SIA DNA is integrated into the chromosomal DNA in a head-to-tail concatemeric manner (right panel).

Immunocytochemical staining of SIA in the liver of
2C. rAAV-LPK-SIA-treated, at 1 month after treatment and
2D. normal control rats. SIA is expressed in the hepatocytes of only the rAAV-LPK-SIA-treated rats.

Histological examination of liver tissue from
2E. rAAV-LPK-SIA-treated, at 1 month after treatment and
2F. normal control rats by hematoxylin and eosin staining.

Immunohistochemical staining of insulin in the pancreatic islet of
2G. rAAV-LPK-SIA-treated rats, at 1 month after treatment and
2H. normal control rats.
2I. Plasma SIA levels were measured at 0 (gray bars) and 4 (black bars) hrs after glucose loading (2 g/kg body weight) at 5, 10, 15, 20 and 25 weeks after rAAV-LPK-SIA treatment (n=7/group).
2J. Plasma C-peptide levels were measured at 0 (gray bars) and 0.5 (black bars) hr after glucose loading at 25 weeks after rAAV-LPK-SIA treatment (n=7). Normal nondiabetic rats (NC; n=7) and untreated STZ-induced diabetic rats(STZ rat; n=7) were used as controls. Values are means ± SD.

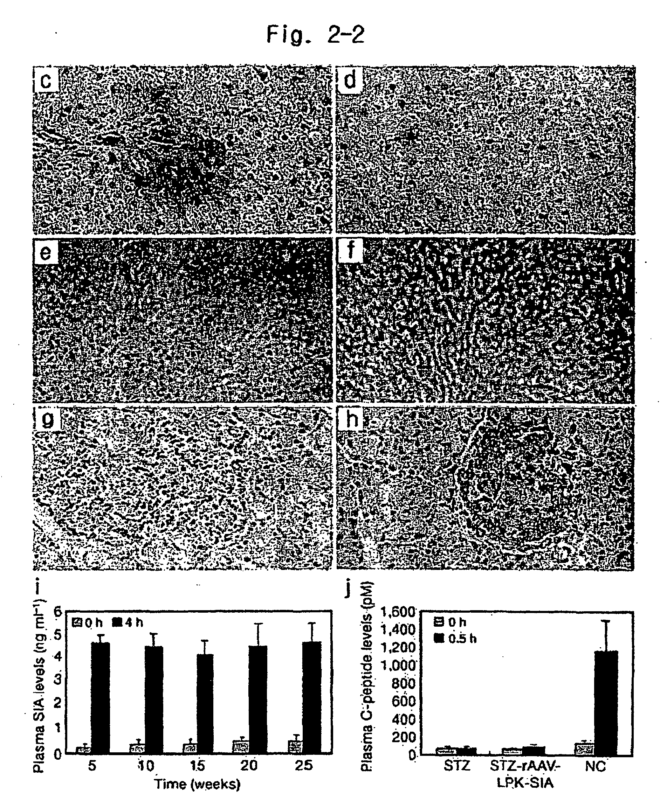

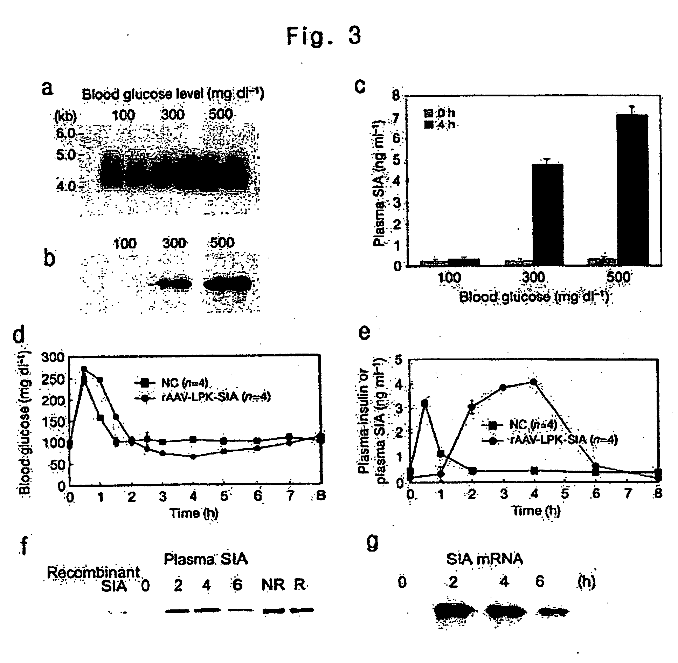

Figures 3A-3G. - The functional response of SIA to glucose in rAAV-LPK-SIA-treated STZ-induced diabetic rats. rAAV-LPK-SIA (1 x 10¹¹ particles) was injected into the hepatic portal vein of STZ-induced diabetic SD rats. At 30 weeks after the treatment, different levels of blood glucose (about 100 (saline-treated), 300 or 500 mg/dl) were maintained for 30 min using a hyperglycemic clamp.
3A. The presence of SIA DNA,
3B. The expression of SIA in the hepatocytes and
3C. The production of SIA in the plasma was determined at 0 (gray bars) and 4 (black bars) hrs (n=7/group). At 4 weeks after treatment, rats (15-17 weeks old) were fasted for 4 hrs and a GTT was performed. Blood samples were collected from the tail vein at the indicated times following the injection of glucose, and levels of:
3D. glucose and
3E. insulin or SIA were measured. (NC), normal control rats; (rAAV-LPK-SIA), rAAV-LPK-SIA-treated rats (1 x 10¹¹ particles). Each time point represents the mean ± SD value.
3F. Western blot analysis of SIA. The release of SIA was examined in plasma collected at 0, 2, 4 and 6 hrs after glucose loading by western blot using anti-SIA antibodies. As a control, the recombinant SIA protein was used. NR, non-reducing conditions; R, reducing conditions.
3G. The expression of SIA mRNA was examined in the liver at 0, 2, 4 and 6 hrs after glucose loading by RNase protection assay.

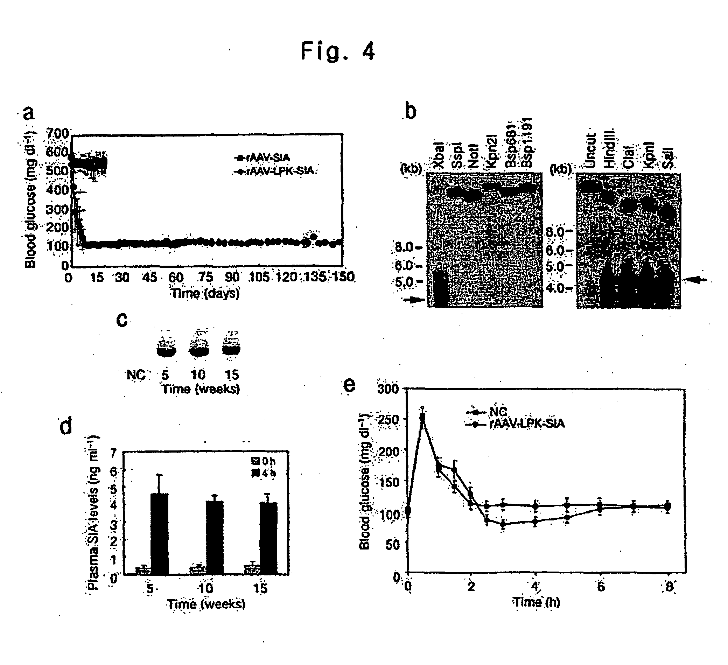

Figures 4A-4E. - Remission of autoimmune diabetes in NOD mice by administration of rAAV-LPK-SIA.
4A. Diabetic NOD mice were administered 1 x 10¹² virus particles of rAAV-SIA or rAAV-LPK-SIA per mouse (n=6/group), and blood glucose was measured.
4B. shows the integration of SIA DNA into the hepatocyte chromosomal DNA. Total cellular DNA was isolated from the livers of NOD mice at 15 weeks after rAAV-LPK-SIA treatment and digested with the indicated restriction enzymes. Southern blot hybridization was performed. The band pattern was the same as that in rAAV-LPK-SIA-treated rats, indicating that rAAV-LPK-SIA DNA is integrated into the chromosomal DNA (arrows, 4.3 Kb band).
4C. The expression of SIA mRNA in hepatocytes (NC, untreated control NOD mice) and
4D. the production of SIA in the plasma was measured at 0 (gray bars) and 4 (black bars) hrs after glucose loading (2 g/kb body weight) at 5, 10 or 15 weeks after rAAV-LPK-SIA treatment (n = 5/group).
4E. GTT test was performed in rAAV-LPK-SIA-treated diabetic NOD mice (n = 5) after the remission of diabetes and in age-matched normal control NOR mice (NC, n = 7). Each time point represents the mean ± SD value.

### DETAILED DESCRIPTION OF THE INVENTION

As used herein, the term "patient" includes members of the animal kingdom including but not limited to human beings.

As used herein, the term "mammalian host" includes members of the animal kingdom including but not limited to human beings.

As used herein, the term "diabetes" is a hormonal disorder. Insulin is needed to control the blood sugar levels.

As used herein, the term "Type I diabetes" means insulin-dependent diabetes mellitus (IDDM).

As used herein, the term "Type II diabetes" means non insulin-dependent diabetes mellitus (NIDDM).

Insulin is composed of two peptide chains referred to as the A chain and B chain. A and B chains are linked together by two disulfide bonds, and an additional disulfide is formed within the A chain. In most species, the A chain consists of 21 amino acids and the B chain of 30 amino acids. Although the amino acid sequence of insulin varies among species, certain segments of the molecule are highly conserved, including the positions of the three disulfide bonds, both ends of the A chain and the C-terminal residues of the B chain. These similarities in the amino acid sequence of insulin lead to a three dimensional conformation of insulin that is very similar among species, and insulin from one animal is very likely biologically active in other species. Indeed, pig insulin has been widely used to treat human patients.

Insulin molecules have a tendency to form dimers in solution due to hydrogen-bonding between the C-termini of B chains. Additionally, in the presence of zinc ions, insulin dimers associate into hexamers.

These interactions have important clinical ramifications. Monomers and dimers readily diffuse into blood, whereas hexamers diffuse very poorly. Hence, absorption of insulin preparations containing a high proportion of hexamers is delayed and slow. This problem, among others, has stimulated development ofa number of recombinant insulin analogs. The first of these molecules to be marketed - called insulin lispro - is engineered such that lysine and proline residues on the C-terminal end of the B chain are reversed; this modification does not alter receptor binding, but minimizes the tendency to form dimers and hexamers.

As used herein, the "single-chain insulin analog (SIA)" encompasses a group of structurally-related proteins wherein the A and B chains are covalently linked by a polypeptide linker. SIA has the properties of greater insulin receptor binding activity and/or glucose uptake activity than proinsulin, and less insulin receptor binding activity and glucose uptake activity than insulin. "SIA-1", "SIA-2" and so on belong to the SIA group.

The polypeptide linker connects the C-terminus of the B chain to the N-terminus of the A chain. The linker is 7 or 9 amino acids long. The most preferred sequence for the linker is Gly-Gly-Gly-Pro-Gly-Lys-Arg or Arg-Arg-Gly-Pro-Gly-Gly-Gly.

It is also to be understood that the insulin A and B chains may be modified or fragmented so long as the modified or fragmented form has glucose uptake activity and/or binds to the insulin receptor, wherein a SIA formed from these chains possesses greater insulin receptor binding activity and/or glucose uptake activity than proinsulin, and less insulin receptor binding activity and glucose uptake activity than insulin.

As used herein, a "promoter" can be any sequence of DNA that is active, and controls transcription in an eucaryotic cell. Preferably, the promoter is active in mammalian cells. The promoter may be constitutively expressed or inducible. Preferably, the promoter is inducible. Preferably, the promoter is inducible by an external stimulus. More preferably, the promoter is inducible by hormones or metabolites: Still more preferably, the promoter is regulatable by glucose. Even more preferably, the promoter is a pyruvate kinase gene promoter. Most preferably, the promoter is a hepatocyte-specific L-type pyruvate kinase gene promoter.

Likewise, "enhancer elements", which also control transcription, can be inserted into the DNA vector construct, and used with the construct of the present invention to enhance the expression of the gene of interest.

As used herein viral vectors include any virus that is useful in *in vivo* or *ex vivo* gene therapy protocols. Preferably, the virus is non-pathogenic. More preferably, the virus is adeno-associated virus (AAV).

As used herein, the term "DC-chol" means a cationic liposome containing cationic cholesterol derivative. The "DC-chol" molecule includes a tertiary amino group, a medium length spacer arm (two atoms) and a carbamoyl linker bond (Gao et al., Biochem. Biophys. Res, Commun., 179:280-285, 1991).

As used herein, "SF-chol" is defined as a type of cationic liposome.

As used herein, the term "biologically active" used in relation to liposomes denotes the ability to introduce functional DNA and/or proteins into the target cell.

As used herein, the term "biologically active" in reference to a nucleic acid, protein, protein fragment or derivative thereof is defined as an ability of the nucleic acid or amino acid sequence to mimic a known biological function elicited by the wild type form of the nucleic acid or protein.

As used herein, the term "maintenance", when used in the context of liposome delivery, denotes the ability of the introduced DNA to remain present in the cell. When used in other contexts, it means the ability of targeted DNA to remain present in the targeted cell or tissue so as to impart a therapeutic effect.

The present invention discloses *in vivo* techniques for delivery of a DNA sequence of interest to the tissue cells of the mammalian host. The *in vivo* technique involves directly administering a DNA vector containing a DNA sequence of interest or other delivery vehicle of interest into the tissue cells, to the target area of the mammalian host, so as to effect *in vivo* expression of the gene product of interest. The vector is preferably a viral vector.

Alternatively, the present invention discloses *ex vivo* and *in vivo* techniques for delivery of a DNA sequence of interest to the tissue cells of the mammalian host. The *ex vivo* technique involves culturing target tissue cells, *in vitro* transfecting a DNA vector containing a DNA sequence of interest or other delivery vehicle of interest into the tissue cells, followed by transplantation of the modified tissue cells to the target area of the mammalian host, so as to effect *in vivo* expression of the gene product of interest.

As an alternative to the *in vitro* manipulation of cells, the gene encoding the product of interest is introduced into liposomes and injected directly into the target area, where the liposomes fuse with the tissue cells, resulting in an *in vivo* gene expression of SIA.

As an additional alternative to the *in vitro* manipulation of tissue cells, the gene encoding the product of interest is introduced into the target area as naked DNA. The naked DNA enters the tissue cell, resulting in an *in vivo* gene expression of SIA.

One *ex vivo* method of treating diabetes comprises initially generating a recombinant viral or plasmid vector which contains a DNA sequence encoding single-chain insulin analog or biologically active fragment thereof This recombinant vector is then used to infect or transfect a population of *in vitro* cultured tissue cells, resulting in a population of cells containing the vector. Expression of this DNA sequence of interest is useful in substantially reducing at least one deleterious pathology associated with diabetes.

More specifically, this method includes employing SIA, or a biologically active derivative or fragment thereof or a biologically active derivative or fragment thereof.

A further embodiment of the present invention includes employing SIA or a biologically active derivative or fragment thereof, and employing as the DNA plasmid vector any DNA plasmid vector known to one of ordinary skill in the art capable of stable maintenance within the targeted cell or tissue upon delivery, regardless of the method of delivery utilized.

One such method is the direct delivery of the DNA vector molecule, whether it be a viral or plasmid DNA vector molecule, to the target cell or tissue. This method also includes employing SIA or biologically active derivative or fragment thereof.

Another embodiment of this invention provides a method for introducing at least one gene encoding a product into at least one cell of a target tissue for use in treating the mammalian host. This method includes employing non-viral means for introducing the gene coding for the product into the tissue cell. More specifically, this method includes a liposome encapsulation, calcium phosphate coprecipitation, electroporation, or DEAE-dextran mediation, and includes employing as the gene a gene capable of encoding a member of transforming growth factor superfamily or biologically active derivative or fragment thereof, or biologically active derivative or fragment thereof.

Another embodiment of this invention provides an additional method for introducing at least one gene encoding a product into at least one cell of a tissue for use in treating the mammalian host. This additional method includes employing the biologic means of utilizing a virus to deliver the DNA vector molecule to the target cell or tissue. Preferably, the virus is a pseudo-virus, the genome having been altered such that the pseudo-virus is capable only of delivery and stable maintenance within the target cell, but not retaining an ability to replicate within the target cell or tissue. The altered viral genome is further manipulated by recombinant DNA techniques such that the viral genome acts as a DNA vector molecule which contains the heterologous gene of interest to be expressed within the target cell or tissue. Preferably, the viral vector is adeno-associated virus.

A preferred method of the present invention involves direct *in vivo* delivery of a SIA gene to the target tissue of a mammalian host through use of an adeno-associated virus (AAV) vector. In other words, a DNA sequence of interest encoding a functional SIA protein or SIA fragment is subcloned into the respective viral vector. The SIA containing viral vector is then grown to adequate titer and directed into the targeted area, preferably by injection into the portal vein.

Direct injection of a DNA molecule containing the gene of interest into the joint results in transfection of the recipient tissue cells and hence bypasses the requirement of removal, *in vitro* culturing, transfection, selection, as well as transplanting the DNA vector containing-cell to promote stable expression of the heterologous gene of interest.

Methods of presenting the DNA molecule to the target tissue includes, but is not limited to, encapsulation of the DNA molecule into cationic liposomes, subcloning the DNA sequence of interest in a retroviral or plasmid vector, or the direct injection of the DNA molecule itself into the targeted area. The DNA molecule is preferably presented as a DNA vector molecule, either as recombinant viral DNA vector molecule or a recombinant DNA plasmid vector molecule. Expression of the heterologous gene of interest is ensured by inserting a promoter fragment active in eukaryotic cells directly upstream of the coding region of the heterologous gene. One of ordinary skill in the art may utilize known strategies and techniques of vector construction to ensure appropriate levels of expression subsequent to entry of the DNA molecule into the targeted tissue.

In a preferred embodiment, tissue cells recovered from the patient are cultured *in vitro* for subsequent utilization as a delivery system for gene therapy. It will be apparent that Applicants are not limited to the use of the specific tissue type or source disclosed. It would be possible to utilize other tissue sources for *in vitro* culture techniques. The method of using the gene of this invention may be employed both prophylactically and in the therapeutic treatment of diabetes.

In another embodiment of this invention, a compound for parenteral administration to a patient in a therapeutically effective amount is provided that contains a gene encoding a SIA protein and a suitable pharmaceutical carrier.

Another embodiment of this invention provides for a compound for parenteral administration to a patient in a prophylactically effective amount that includes a gene encoding a SIA protein and a suitable pharmaceutical carrier.

A further embodiment of this invention includes the method as hereinbefore described including introducing the gene into the cell *in vitro.* This method also includes subsequently transplanting the infected cell into the mammalian host. This method includes after effecting the transfecting of the tissue cell but before the transplanting of the infected cell into the mammalian host, storing the transfected tissue cell. It will be appreciated by those skilled in the art that the infected tissue cell may be stored frozen in 10 percent DMSO in liquid nitrogen. This method includes employing a method to substantially prevent the development of diabetes in a mammalian host having a high susceptibility of developing diabetes.

Another embodiment of this invention includes a method of introducing at least one gene encoding a product into at least one cell of a target tissue of a mammalian host for use in treating the mammalian host as hereinbefore described including effecting *in vivo* the infection of the cell by introducing the viral vector containing the gene coding for the product directly into the mammalian host. Preferably, this method includes effecting the direct introduction into the mammalian host by injection through the portal vein. This method includes employing the method to substantially prevent the development of diabetes in a mammalian host having a high susceptibility of developing diabetes. This method also includes employing the method on a diabetic mammalian host for therapeutic use.

In a preferred embodiment, the inventors generated a single chain insulin analog (SIA), which possesses biologically active insulin activity without processing and produced a recombinant adeno-associated virus expressing SIA (rAAV-LPK-SIA) under the control of hepatocyte-specific L-type pyruvate kinase gene promoter which regulates SIA expression depending on the level of blood glucose. When the inventors administered rAAV-LPK-SIA through the portal vein of streptozotocin (STZ)-induced diabetic rats, the blood glucose levels decreased, reaching the level of normoglycemia in 1 week and maintained a normoglycemic state for more than 6 months without hypoglycemia or any apparent side effects. In addition, the treatment of diabetic NOD mice with rAAV- LPK-SIA resulted in the complete remission of autoimmune diabetes as seen in STZ-induced diabetes. This novel SIA gene therapy is believed to have therapeutic value for the cure of autoimmune diabetes in humans.

The following examples are offered by way of illustration of the present invention, and not by way of limitation.

### EXAMPLES

### EXAMPLE 1 - MATERIALS AND METHODS

### Cloning and expression of single chain insulin analog (SIA) DNA in E. coli:

SIA-1 DNA encoding Gly-Gly-Gly-Pro-Gly-Lys-Arg sequence in the linker region of SIA was generated by polymerase chain reaction (PCR) using five overlapping oligonucleotides of 65-68 bases in length. The SIA-1 DNA was constructed by considering the codon usage of *E*. *coli* to increase the expression level of SIA in the bacterial hosts. The resulting SIA-1 DNA sequence was:
ATG/TTC/GTT/AAT/CAG/CAC/CTG/TGC/GGC/TCT/CAC/CTG/GTA/GAA/GCT/CTG/T AC/CTG/GTT/TGC/GGT/GAA/CGT/GGT/TTT/TTC/TAC/ACC/CCG/AAA/ACC/GGT/GG T/GGT/CCG/GGT/AAA/CGT/GGC/ATC/GTT/GAA/CAA/TGC/TGT/ACT/AGC/ATC/TG C/TCT/CTC/TAC/CAG/CTG/GAG/AAC/TAT/TGT/AAC/TAG/TAA. The N-terminal pentapeptide sequence (PSDKP) of TNF-α was used as a fusion partner to produce SIA-1 with high-level expression in *E*. *coli*. For convenience in the purification process, 10 histidine residues and a methionine residue for chemical cleavage were inserted between the PSDKP sequence and SIA-1. A DNA fragment encoding the PSDKP sequence and 10 histidine residues was chemically synthesized. After digestion with restriction endonucleases *Nde*I and *Bam*HI, the DNA fragment was inserted downstream of the T7 promoter of the expression plasmid pET-3a, which was linearized with the same restriction endonucleases, and the resulting plasmid was named pET. The gene encoding SIA-1 was digested with *Bam*HI and *Hin*dIII and inserted into the *Bam*HI and *Hin*dIII enzyme restriction sites of the pET plasmid and the resulting plasmid was named pET-SIA-1. This expression plasmid was then used to transform *E*. *coli* BL21 (DE3) cells, and the fused single chain insulin analog was expressed as inclusion bodies. The inclusion bodies of the fusion protein were sulfonated at their cysteine residues and chemically cleaved by CNBr treatment. S-sulfonated SIA-1 was purified by cation-exchange chromatography (Pharmacia Biotechnology) and refolded by addition of β-mercaptoethanol and analyzed by analytical reverse-phase HPLC. Briefly, sulfonated SIA-1 (0.37 mg/ml) was converted to SIA-1 with native disulfide pairings in 50 mM glycine buffer, pH 11.0, at 4°C using 2 equivalents of β-mercaptoethanol. After 20 hr, the protein solution was acidified to pH 2.5 to terminate the reaction, loaded onto a Zorbax C8 column and eluted with a linear gradient of 90% acetonitrile. The fractions containing the desired material were pooled, frozen and lyophilized.

Other SIA DNAs encoding Arg-Arg-Gly-Pro-Gly-Gly-Gly, Gly-Gly-Gly-Gly-Gly-Lys-Arg, Arg-Arg-Gly-Gly-Gly-Gly-Gly, Gly-Gly-Ala-Pro-Gly-Asp-Val-Lys-Arg, Arg-Arg-Ala-Pro-Gly-Asp-Val-Gly-Gly, Gly-Gly-Tyr-Pro-Gly-Asp-Val-Lys-Arg, Arg-Arg-Tyr-Pro-Gly-Asp-Val-Gly-Gly, Gly-Gly-His-Pro-Gly-Asp-Val-Lys-Arg and Arg-Arg-His-Pro-Gly-Asp-Val-Gly-Gly sequence in the linker region of SIA, respectively, were prepared by PCR using the SIA-1 gene as a template DNA. These genes were digested with *Bam*HI and *Hin*dIII and inserted into plasmid pET. The resulting plamids were named pET-SIA-2, pET-SIA-3, pET-SIA-4, pET-SIA-5, pET-SIA-6, pET-SIA-7, pET-SIA-8 and pET-SIA-9, respectively. These expression plasmids were then used to transform *E*. *coli* BL21 (DE3) cells, and the fused SIAs were expressed as inclusion bodies. The purification processes for SIAs were essentially the same as that of SIA-1.

### Examination of functional activity of recombinant SIA produced in E. coli.

Insulin receptor binding and glucose uptake assays were performed using IM-9 lymphocytes as described previously (Pollet, R. J., Standaert, M. L. & Haase, B. A. Insulin binding to the human lymphocyte receptor. Evaluation of the negative cooperativity model. *J*. *Biol*. *Chem*. 252, 5828-5834 (1977); Roth, J. Assay of peptide hormones using cell receptors: application to insulin and to human growth hormone. *Methods Enzymol*. 37, 66-82 (1975); and Frost, S. C. & Lane, M. D. Evidence for the involvement of vicinal sulfhydryl groups in insulin-activated hexose transport by 3T3-L1 adipocytes. *J*. *Biol*. *Chem.* 260, 2646-2652 (1985)). To study the *in vivo* hypoglycemic activity of SIA, 8-10 week-old male Sprague-Dawley rats at 200g to 250 g in body weight, as described previously (Heath, W. F., *et al*. (A-C-B) human proinsulin, a novel insulin agonist and intermediate in the synthesis of biosynthetic human insulin. *J*. *Biol*. *Chem*. 267, 419-425 (1992)), were fasted, and SIA protein (4 to 80 µg in 0.1 ml saline/100 g body weight) or the same volume of saline as a control was injected subcutaneously. Blood was obtained from the tail vein of each rat and the glucose level was determined before (time zero) and 30 min, 1, 2, 3 and 4 h after administration of SIA. The mean percentage change in the blood glucose level from time zero of the saline- and SIA-treated rats was calculated and the final results were expressed after adjusting for the change in the control group. The effect of different doses of each peptide (4 to 80 µg) was determined. ED₅₀ values are presented as the dose of the protein resulting in one-half of the maximum hypoglycemic activity at 1 or 2 hr after SIA administration.

### Construction of pSIA and pLPK-SIA

The SIA cDNA from pET-SIA was subcloned into the PCR-script sk⁺ (Invitrogen, San Diego, CA) at the BamHI/HindIII site. Then the SV40 poly(A) signal sequence from pCDM8 (Invitrogen) and the SV40 enhancer from the pGL3 (Promega, Madison, WI) were amplified by PCR and subcloned at the HindIII/ApaI and Apal sites, respectively. The albumin leader sequence (72 base pairs) was inserted in front of the SIA cDNA using a ExSite™ PCR-based Site-Directed Mutagenesis Kit (Stratagene, La Jolla, CA) using the following primer set: the 5' primer (63 mer) containing a sequence complementary to the upstream 27 nucleotides of the SIA cDNA and the 5' 36 nucleotides of the human albumin leader sequence (TTAGCTCGGC TTATTCCAGG GGTGTGTTTC GTCGAGATTT CGTTAATCAG CACCTGTGCG GCT) and the 3' primer (63 mer) containing the 3' 36 nucleotides of the albumin leader sequence and the 27 nucleotides of the SIA cDNA (AGAGAAAAAG AAGGGAAATA AAGGTTACCC ACTTCATGGA TCCGCCCAGT CGTCGACGCT GCT). The clone containing the albumin leader sequence was isolated and designated as pSIA. The final construct, pLPK-SIA, was generated by insertion of the promoter of the rat LPK gene (-3193 to +18) amplified by PCR into pSIA at the XbaI/SalI site.

### Administration of pSIA, pLPK-SIA, rAAV-SIA or rAAV-LPK-SIA into the liver.

SD rats or NOD mice were anaesthetized with Ketamine-chloride (10 mg/kg) and ether. A midline abdominal incision was made, and 30 µg of DNA-FuGENE™ 6 (Boehringer Mannheim GmBH, Laval PQ) mixture, rAAV-SIA, or rAAV-LPK-SIA (10⁹ - 10¹² virus particles) was injected into the hepatic portal veinofNOD mice or 11-13 week-old SD rats that had been in the hyperglycemic state for 2 weeks.

### PCR and RT-PCR analysis:

To examine the presence of SIA DNA in plasmid-injected rats, PCR was performed using the sense primers derived from the T7 or LPK promoter region (5' GTAATACGACTCACTATAG GGC 3' for pSIA-injected rats; 5' ATTTCGAATAAGAAGAGGAAGGGAAG 3' for pLPK-SIA-injected rats) and the antisense primers derived from the 3' terminus of the SIA gene (5' GCGCAAGCTTTTACTAGTTACAATAGTT 3'). To detect SIA mRNA, the total RNA was isolated form various tissues and RT-PCR was performed using the primers 5' GCGCGGATCCATGTTCGTTAATCAGCAC 3' and 5' GCGCAAGCTTTTACTAGTTACAATAGTT 3'. β-actin mRNA was amplified as an internal control.

### Production of recombinant AAV-SIA and AAV-LPK-SIA.

The 4.3 Kb LPK-SIA including the SV40 enhancer was amplified by PCR from the pLPK-SIA plasmid and subcloned into psub201 at the XbaI site. The resulting plasmid was designated as psub201-LPK-SIA. Human embryonic kidney 293 cells (ATCC CRL 1573, Manassas, VA), grown in 15 cm dishes, were cotransfected with helper plasmid pXX2 and psub201-LPK-SIA using Lipofectamine plus reagent (Gibco-BRL) according to the manufacturer's protocol. The psub 201-SIA, which does not contain the LPK promoter, was used for the contruction of rAAV-SIA. After 6 h, the transfection medium was replaced with Iscove's modified Dulbecco's medium and the cells were infected with adenovirus type 5 d1312 at a multiplicity of infection (MOI) of 2. Three days after infection, the cells were harvested in HEPES buffer (140 mM NaCI, 25 mM HEPES, 0.7 mM Na₂HPO₄, pH 7.05) and lysed by three cycles of freezing and thawing. The cell lysate was centrifuged at 2,000 x g for 20 min to remove cell debris. The recombinant virus (rAAV-SIA and rAAV-LPK-SIA) was purified through two rounds of cesium chloride equilibrium density gradients to remove any contaminating proteins. Purified rAAV-SIA or rAAV-LPK-SIA stock was heated at 56° C for 45 min to inactivate residual adenoviral particles. For estimation of the number of rAAV-SIA or rAAV-LPK-SIA viral particles, each stock was treated with DNaseI and encapsulated viral DNA was extracted with phenol-chloroform and precipitated with ethanol. The amount of viral DNA was quantitated by competitive PCR (Muzyczka, N. Use of adeno-associated virus as a general transduction vector for mammalian cells. *Curr*. *Top*. *Microbiol*. *Immunol*. 158, 97-129 (1992)).

### Southern blot analysis.

Total cellular DNA (10 µg) was isolated from the livers of normal control or rAAV-LPK-SIA-treated animals and digested with various restriction enzymes. After agarose gel electrophoresis, the DNA was transferred to a membrane and hybridized with a ³²P-labeled probe containing the SIA cDNA and the SV40 sequence. The band was detected by autoradiography.

### Measurement of plasma insulin, C-peptides, SIA and blood glucose.

Plasma insulin and C-peptide levels were measured using the Linco rat insulin RIA kit and Linco rat C-peptide RA kit, respectively (Linco Research Immunoassy, St. Charles, MO). For the measurement of plasma SIA level, the inventors performed competitive ELISA using anti-SIA antibodies raised in rabbit, since SIA does not cross-react well with commercial anti-insulin antibodies. Blood glucose levels were measured or described elsewhere (Yoon , J. W., Lesniak, M.A., Fussganger R. & Notkins, A.L., "Genetic Differences In Suscepibitiliy Of Pancreatic β-Cells To Virus-Induced Diabetes Mellitus, *Nature*, 264, 178-180, (1976)).

### Hyperglycemic clamp experiment

STZ-induced diabetic rats were treated with 10¹¹ particles of rAAV-LPK-SIA. At 30 weeks after the treatment, a 20% glucose solution, for maintenance of blood glucose at about 300 or 500 mg/dl, or saline, for maintenance of blood glucose at about 100 mg/dl, was perfused into the femoral vein using an electronic digital syringe pump (Harvard, South Natick, MA). After an equilibration period, blood glucose levels were determined immediately from the tail vein every 2 min, and the infusion rate was appropriately adjusted to maintain blood glucose levels at either approximately 100, 300, or 500 mg/dl for 30 min. The production of SIA in the hepatocytes and plasma was examined 4 hrs after glucose loading or saline treatment. Hyperglycemic clamp experiment is described in Cameron, N.E., Cotter, M.A. & Low, P.A., "Nerve Blood Flow In Early Experimental Diabetes In Rats: Relation To Conduction Deficits", *AM*. *J*. *Physiol,* 261, E1-E8, (1991).

### Western blot analysis.

The proteins below 30,000 m.w. were separated from 1.5 ml of respective pooled plasma at 0, 2, 4 and 6 h with centricon-30 (Amicon, Beverly, MA) and concentrated using a freeze-dryer. The concentrated proteins, with or without β-mercaptoethanol treatment, were electrophoretically separated on 10-20% tricine gradient gel (NOVEX, San Diego, CA) and transferred to nitrocellulose membrane. The SIA protein bands on blots were visualized with rabbit polyclonal anti-SIA antibody.

### RNase protection assay.

The [³²P]-labeled SIA antisense RNA was made from pET-SIA by *in vitro* transcription using T7 RNA polymerase. Ten µg of total RNA isolated from the livers of rAAV-LPK-SIA-treated animals was hybridized with S x 10⁵ cpm of labeled probes. After RNase treatment, the protected probes were resolved on a 6 % denaturing polyacrylamide gel and autoradiography was performed.

### Measurement of AST and ALT.

AST and ALT were measured in an autochemical analyzer (Hitachi 747, Tokyo, Japan) using an ultraviolet assay method (Ma, Z., *et al*. Effect of hemoglobin- and Perflubron-based oxygen carriers on common clinical laboratory tests. *Clin*. *Chem*. 43, 1732-1737 (1997)).

### Histological and immunocytochemical analysis.

The liver and pancreas were removed, fixed in formalin and stained with hematoxylin and eosin to demonstrate general morphology (Yoon, J. W., Rodrigues, M. M., Currier, C. & Notkins, A. Long-term complications of virus-induced diabetes mellitus in mice. *Nature* 296, 566-569 (1982); and Yoon, J. W. *et al*. Control of autoimmune diabetes in NOD mice by GAD expression or suppression in cells. *Science* 284, 1183-1187 (1999)). Sections of liver and pancreas were also incubated with anti-SIA (made in rabbits) or anti-human insulin antibodies, respectively, and immunostained using a labeled streptavidin-biotin method (Dakopatts A/S, Glostrup, Denmark) and visualized by 3'-amino-9-ethylcarbazole (Dako, Santa Barbara, CA, U.S.A) (Yoon, J. W. *et al*. Control of autoimmune diabetes in NOD mice by GAD expression or suppression in cells. *Science* 284, 1183-1187 (1999); and Hirasawa K. *et al*. Possible role of macrophage-derived soluble mediators in the pathogenesis of EMC virus-induced diabetes in mice, *J Virol*. 71,4024-4031 (1997)).

### EXAMPLE 2 - RESULTS

The development of Type 1 diabetes, also known as insulin-dependent diabetes mellitus (IDDM), results from the almost total destruction of insulin-producing pancreatic β cells by β cell-specific autoimmune responses (Yoon, J. W. & Jun, H. S. Insulin-dependent diabetes mellitus. In: Roitt, I. M. & Delves, P. J. eds. *Encyclopedia of Immunology, Second Edition*. London, UK: Academic Press Ltd. pp. 1390-1398 (1998); Schranz, D. B. & Lernmark, A. Immunology in diabetes: an update. *Diab*. *Metab. Rev*. 14: 3-29 (1998); Tisch, R. & McDevitt, H. Insulin-dependent diabetes mellitus. *Cell* 85: 291-297 (1996); Bach, J. F. Insulin-dependent diabetes mellitus as a β cell targeted disease of immunoregulation. *J*. *Autoimmunity* 8: 439-463 (1995); and Rossini, A. A., Greiner, D. L., Friedman, H. P. & Mordes, J. P. Immunopathogenesis of diabetes mellitus. *Diabetes Rev.* 1: 43-75, (1993)). Standard insulin therapy may not maintain blood glucose concentrations within the relatively narrow range that occurs in normal pancreatic β cells (The Diabetes Control and Complications Trial Research Group. The effect of intensive treatment of diabetes on the development and progression of long-term complications in insulin-dependent diabetes mellitus. *New Engl*. *J*. *Med*. 329, 977-986 (1993)). The inventors have approached the permanent cure of IDDM by means of a novel gene therapy using a single-chain insulin analog, which possesses biologically active insulin activity without processing, and a glucose regulatable promoter, hepatocyte-specific L-type pyruvate kinase (LPK) promoter.

First, the inventors generated a single-chain insulin analog (SIA-1) by replacing 35 residues of the C-peptide with a short turn-forming heptapeptide (Gly-Gly-Gly-Pro-Gly-Lys-Arg). The inventors produced recombinant SIA-1 in *Escherichia coli*, refolded it, and examined its biological activity using receptor binding and glucose uptake assays. The inventors found that the receptor binding activity of SIA-1 was 12-fold higher than that of proinsulin and 3- to 4-fold lower than that of insulin. Similarly, the glucose uptake activity of SIA-1 was 16-fold higher than that of proinsulin and 4- to 5-fold lower than that of insulin (Table 1). To determine whether SIA has a sufficient capability to control blood glucose in animals, as does insulin, the inventors administered SIA to 8 week-old Sprague-Dawley (SD) rats and determined the concentration of glucose in the whole blood. The inventors found that the hypoglycemic effect of SIA was 2- to 3-fold higher than that of proinsulin and 2-fold lower than that of insulin (Table 1). This result indicates that the biological activity of the recombinant SIA is somewhat comparable to that of insulin.

Second, the inventors constructed a recombinant plasmid, pLPK-SIA (Fig. 1A) by cloning the SIA gene under the LPK promoter (Cuif, M. H., Doiron, B. & Kahn, A. Insulin and cyclic AMP act at different levels on transcription of the L-type pyruvate kinase gene. *FEBS Lett*. 417, 81-84 (1997); Chen, R., Doiron, B. & Kahn, A. Glucose responsiveness of a reporter gene transduced into hepatocytic cells using a retroviral vector. *FEBS Lett*. 365, 223-226 (1995); Decaux, J. F., Antoine, B. & Kahn, A. Regulation of the expression of the L-type pyruvate kinase gene in adult rat hepatocytes in primary culture, *J*. *Biol*. *Chem*. 264, 11584-11590 (1989); Cuif, M. H., Porteu, A., Kahn, A. & Vaulont, S. Exploration of a liver-specific, glucose/insulin-responsive promoter in transgenic mice. *J*. *Biol*. *Chem*. 268, 13769-13772 (1993); and Bergot, M. O., Diaz-Guerra, M. J., Puzenat, N., Raymondjean, M. & Kahn, A. Cis-regulation of the L-type pyruvate kinase gene promoter by glucose, insulin and cyclic AMP. *Nucleic Acids Res*. 20, 1871-1877 (1992)), which will regulate SIA gene expression depending on the level of blood glucose. The albumin leader sequence (24 amino acids) was added to the frame of the SIA gene construct to facilitate the secretion of SIA from the hepatocytes. To determine whether pLPK-SIA can control IDDM by inducing the proper secretion of biologically active insulin analogue, the inventors mixed the pLPK-SIA with FuGENE™ 6 and administered it into the portal vein of streptozotocin (STZ)-induced diabetic SD rats. The inventors found that the blood glucose levels gradually decreased in the pLPK-SIA-injected rats until the fifth day after the administration and remained in a normoglycemic state for an additional 4 days. However, the blood glucose levels of the pLPK-SIA-injected rats gradually increased to reach 500 mg/dl at 14 days after treatment (Fig. 1B). In contrast, the blood glucose levels were not changed in the STZ-induced diabetic rats that were administered with the SIA construct without pLPK (Fig. 1B). To determine whether the plasmid DNA was specifically transfected into the liver and whether the SIA mRNAs were selectively expressed in the liver cells, the inventors examined the presence of the injected DNA and the expression of SIA mRNA in various organs including the liver, kidney, spleen, lung and heart from both groups of the rats at 5 days after the plasmid administration. The inventors found that the transfected plasmid DNAs were detected in only liver cells from both groups of rats, whereas SIA mRNA was expressed in only the pLPK-SIA-treated rats (Fig. 1C). Through these studies, the inventors found that SIA mRNA was exclusively expressed in the liver of the pLPK-SIA-treated rats, the treatment of diabetic rats with pLPK-SIA resulted in the transient remission of IDDM, and hyperglycemia recurred within 14 days after treatment probably due to the short half-life of the transfected pLPK-SIA DNA in the rat.

Third, the inventors attempted to overcome the short half-life of transfected DNA using adeno-associated virus (AAV) vector, since it has been shown to be a safe and efficient gene delivery system (Muzyczka, N. Use of adeno-associated virus as a general transduction vector for mammalian cells. *Curr*. *Top. Microbiol*. *Immunol*. 158, 97-129 (1992); and Clark, K. R., Liu, X., McGrath, J. P. & Johnson, P. R. Highly purified recombinant adeno-associated virus vectors are biologically active and free of detectable helper and wild-type viruses. *Human Gene Therapy* 10, 1031-1039 (1999)), and integrates into the host chromosomal DNA in a site-specific manner (Samulski, R. J. Adeno-associated virus: integration at a specific chromosomal locus. *Curr*. *Opin*. *Genet. Dev*. 3, 74-80 (1993); Giraud, C., Winocour, E. & Berns, K. I. Site-specific integration by adeno-associated virus is directed by a cellular DNA sequence. *Proc*. *Natl*. *Acad*. *Sci. USA* 91, 10039-10043 (1994); and Kotin, R. M., Linden, R. M. & Berns, K. I. Characterization of a preferred site on human chromosome 19q for integration of adeno-associated virus DNA by non-homologous recombination. *EMBO J*. 11, 5071-5078 (1992)). The inventors produced recombinant AAV containing LPK promoter-SIA DNA (rAAV-LPK-SIA) in 293 cells. To determine whether rAAV-LPK-SIA can efficiently and permanently control IDDM, the inventors administered rAAV-LPK-SIA through the portal vein of STZ-induced diabetic SD rats. The blood glucose levels gradually decreased in the rAAV-LPK-SIA-treated rats (10¹¹ virus particles/rat), reached the level of normoglycemia one-week after treatment, and remained in a normoglycemic state for more than 8 months without hypoglycemic or any apparent side effects (Fig. 2A). However, lower doses of rAAV-LPK-SIA (10⁹-10¹⁰ virus particles) appeared to be insufficient for complete remission. To determine whether the treatment of diabetic rats with a dose higher than the curable optimum dose (10¹¹ virus particles of rAAV-LPK-SIA) might result in hypoglycemia, the inventors administered a 10-fold higher dose of rAAV-LPK-SIA (10¹² virus particles of rAAV-LPK-SIA). None of the treated animals became hypoglycemic, indicating that the expression of SIA under the LPK promoter is properly regulated by the level of blood glucose. The body weight of the animals treated with 10¹¹ virus particles of rAAV-LPK-SIA showed body weight similar to normal rats.

The inventors then attempted to find the molecular state of the AAV genome in the recipients of rAAV-LPK-SIA. The inventors removed livers from rats treated with the curable optimum dose of rAAV-LPK-SIA at 5 days, 5 weeks, 15 weeks and 25 weeks after treatment, extracted the DNA and performed southern blot with SIA cDNA and the SV40 sequence as a probe. The inventors found that rAAV DNA exists in both single-stranded and double-stranded states at the early stage (5 days) after the treatment, but found only the double-stranded state at a later stage (5-25 weeks) after the treatment. When the inventors examined the integration of rAAV-LPK-SIA DNA in the hepatocyte DNA at 25 weeks after the treatment. rAAV-LPK-SIA DNA was found to be integrated into the chromosomal DNA in a head-to-tail concatemeric manner (Fig. 2B). These results indicate that the single-stranded recombinant adeno-associated viral genome is converted to a double-stranded genome in the infected hepatocytes.

The inventors also determined the localization of SIA-expression in the liver of rats treated with rAAV-LPK-SIA by immunohistochemical staining of liver sections with anti-SIA antibody. The inventors found that SIA is expressed in the hepatocytes of the liver from the rAAV-LPK-SIA-treated rats (Fig. 2C), but not in the liver from untreated control rats (Fig. 2D). SIA-expressing cells were clustered around the central vein and portal triads in the liver (Fig. 2C). To determine whether infection of hepatocytes with rAAV-LPK-SIA results in damage to the infected cells, the inventors histologically examined the infected liver sections. The inventors found that there was no difference between untreated rats and rAAV-LPK-SIA-treated rats (Figs. 2E and 2F). The inventors then determined whether prolonged hepatic SIA expression causes any other liver damage by liver function tests. The inventors found no difference in the level of plasma aspartate transaminase (AST) or plasma alanine transaminase (ALT), marker enzymes for hepatic damage, between saline-treated control rats (94 ± 12 IU/l for AST and 46 ± 9 IU/l for ALT; n=5) and rAAV-LPK-SIA-treated rats (95 ± 10 IU/l for AST and 40 ± 9 IU/l for ALT), indicating that rAAV-LPK-SIA treatment does not cause liver damage. The inventors also examined the development of antibodies against SIA every five weeks until 8 months after treatment and found that anti-SIA antibody was barely detected in the sera of the treated rats (data not shown). These results indicate that treatment of diabetic rats with rAAV-LPK-SIA resulted in the remission of diabetes without any apparent change in the infected liver cells or any adverse effects of SIA expression on the hepatocytes.

Then, the inventors questioned whether the remission of diabetes might be due to the secretion of insulin from the residual pancreatic β cells in the STZ-treated rats, rather than the expression of SIA. The inventors stained pancreatic sections from rAAV-LPK-SIA-treated rats with anti-insulin antibody, and found that insulin-producing β cells were rarely found in these islets (Fig. 2G) whereas abundant insulin-producing β cells were found in the islets of untreated normal rats (Fig. 2H). When SIA levels in the plasma of the rats were examined every 5 weeks after treatment, it was found that SIA was continuously produced for over 8 months after the treatment with rAAV-LPK-SIA (Fig. 2I; data after 25 weeks not shown). In addition, the C-peptide level in the rAAV-LPK-SIA-treated rats after glucose loading was measured. A negligible amount of C-peptide was found in plasma of the rats, whereas a substantial amount of C-peptide was found in plasma of nondiabetic normal rats (Fig 2J). These results indicate that the control of blood glucose in rAAV-LPK-SIA-treated rats was not due to endogenous pancreatic insulin, but to the expression of SIA in the liver cells.

Fourth, the inventors questioned whether the expression of SIA is truly regulated by blood glucose levels through the LPK promoter. A 20% glucose solution or saline was perfused into the femoral vein using an electronic digital syringe pump (Cameron, N. E., Cotter, M.A. & Low, P.A., "Nerve Blood Flow In Early Experimental Diabetes In Rats: Relation To Conduction Deficits", *AM*. *J*. *Physiol*, 261, E1-E8, (1991)), and maintained different levels of blood glucose (approximately 100, 300, or 500 mg/dl) for 30 min in rats in which the blood glucose had been normalized after treatment with 10¹¹ particles of rAAV-LPK-SIA, and examined the production of SIA in the hepatocytes and plasma at 4 hrs after glucose loading. The inventors found that the level of SIA expression was closely correlated with the concentration of blood glucose (Figs. 3B and 3C), even though similar levels of SIA DNA were found among the different groups of rats (Fig. 3A), indicating that the expression of SIA under the LPK promoter is properly controlled by blood glucose levels.

Next, the inventors determined whether rAAV-LPK-SIA-treated rats (15-17 weeks old) clear glucose from the blood in.a manner similar to nondiabetic normal rats by glucose tolerance tests (GTT). The inventors injected glucose (2 g/kg body weight i.p.) into rAAV-LPK-SIA-treated rats, which had recovered from diabetes, after four hours of fasting and examined the blood glucose, plasma insulin and glucagon levels at different times after glucose loading. The inventors found that the blood glucose levels of normal rats peaked at 30 min after glucose loading, returned to the normal range (around 100 mg/dl) at 90 min, and stabilized thereafter (Fig. 3D). The blood glucose levels of rAAV-LPK-SIA-treated rats showed a similar pattern as compared to normal rats, except for a slightly delayed recovery time to reach normal glucose levels (120 min vs. 90 min) and transient by lower blood glucose levels from 3 to 6 hrs after glucose loading 3D). The plasma insulin levels of normal rats rapidly peaked within 30 min and returned to basal levels at 2 hrs after glucose loading (Fig. 3E). However, the plasma SIA levels of rAAV-LPK-SIA-treated rats peaked at 3-4 hrs and returned to basal levels at 6 hrs after glucose loading (Figs. 3E and 3F). The expression of SIA mRNA in the hepatocytes showed a similar pattern to that of plasma SIA (Fig. 3G). The response of insulin to glucose in normal rats was very rapid, since insulin is released in normal pancreatic β cells through the process of exocytosis. Although the blood glucose levels of normal and rAAV-LPK-SIA-treated rats were relatively similar, the plasma SIA levels from 2 to 5 hours after the glucose loading were significantly higher than the plasma insulin levels of normal rats. This is probably due to the delayed metabolic processing of SIA, resulting in its longer half-life in the circulation. The slightly lower blood glucose levels in rAAV-LPK-SIA-treated rats from 3 to 6 hr after glucose loading may be due to this prolonged half-life of SIA. As a matter of fact, the secretion of SIA in rAAV-LPK-SIA-treated rats is controlled at the transcriptional level, and thus a prolonged period of time is required to change the plasma SIA levels in response to blood glucose levels, resulting in the prolonged secretion of SIA during GTT in rAAV-LPK-SIA-treated rats. In contrast, insulin is released in normal pancreatic β cells through the process of exocytosis so the insulin response to glucose was rapid in normal cells. The major physical determinant of insulin secretion in the β cells is the concentration of blood glucose. β cells are very sensitive to small changes in extracellular glucose within a narrow physiological range. Thus, the plasma insulin level in normal rats is concomitantly increased with the blood glucose level during GTT. However, the plasma SIA levels in rAAV-LPK-SIA-treated rats are not tightly correlated with the blood glucose levels after glucose loading. Nevertheless, the non-fasting blood glucose levels of rAAV-LPK-SLA-treated rats were always within the normoglycemic range, implying that the expression of SIA is efficiently regulated by the level of glucose. In addition, the inventors measured the level of plasma glucagon in rAAV-LPK-SIA-treated rats, and found that it increased significantly at 6 hours after glucose loading. The increase in glucagon levels correlates with the decrease in plasma levels of SIA and its mRNA (Figs. 3B, 3C and 3E), suggesting that functional α-cells in the pancreatic islets are required for negative feedback regulation on the LPK promoter-mediated SIA expression in rAAV-LPK-SIA-treated rats.

Fifth, the inventors attempted to find whether rAAV-LPK-SIA can also permanently control autoimmune diabetes in non-obese diabetic (NOD) mice as in STZ-induced diabetes in rats. The inventors administered rAAV-LPK-SIA into diabetic NOD mice by intraportal injection and monitored the blood glucose levels. The blood glucose levels gradually decreased in the rAAV-LPK-SIA-treated NOD mice (10¹² virus particles), reached the level of normoglycemia at 7 days after treatment, and remained in a normoglycemic state for more than 5 months. In contrast, diabetic NOD mice treated with rAAV-SIA (without the LPK promoter) remained hyperglycemic and died within 3 weeks (Fig. 4A). When the presence of the SIA gene in the hepatocytes of NOD mice were examined at 15 weeks after treatment with rAAV-LPK-SIA, it was discovered that SIA DNA was integrated into the chromosomal DNA (Fig. 4B). SIA expression was then examined in the liver and plasma of the mice after glucose loading at 5, 10, and 15 weeks after treatment with rAAV-LPK-SIA, and found that SIA mRNA was clearly expressed and SIA protein was released in the plasma (Figs. 4C and 4D). Anti-SIA antibody was not detected in the sera from these mice during the 5 months after treatment (data not shown).

In addition, the inventors performed GTT in NOD mice which had recovered from diabetes. The inventors found that the blood glucose levels of rAAV-SIA-treated NOD mice peaked at 30 min after glucose loading, returned to the normal range (115 mg/dl at 120 min) and stabilized thereafter (Fig. 4E). The time of the peak blood glucose levels in these mice was similar to that found in normal ICR mice, but there was a delay in the recovery time to reach normal blood glucose levels and slightly lower blood glucose levels from 3 to 6 hours after glucose loading as compared with normal controls, as seen in the rAAV-SIA-treated rats.

The inventors have developed a potential method for the treatment of autoimmune type 1 diabetes by the expression of a single-chain insulin analog in the hepatocytes under the control of hepatocyte-specific glucose regulatable promoter and optionally an SV40 enhancer. The host's cell-mediated autoimmune responses do not attack the SIA-expressing hepatocytes, resulting in the permanent remission of autoimmune diabetes in NOD mice. The treatment of both chemically induced diabetes in rats and autoimmune diabetes in NOD mice with rAAV expressing the insulin analog resulted in the permanent remission of type 1 diabetes without any detectable adverse effect on the hepatocytes, suggesting that this novel gene therapy may have therapeutic value for the cure of type 1 diabetes in humans.

### REFERENCES

1. Levine, F. & Leibowitz, G. Towards gene therapy of diabetes mellitus, *Mol*. *Med*. *Today* 5, 165-171 (1999).
2. Yoon, J. W. & Jun, H. S. Insulin-dependent diabetes mellitus. In: Roitt, I. M. & Delves, P. J. eds. *Encyclopedia of Immunology, Second Edition.* London, UK: Academic Press Ltd. pp. 1390-1398 (1998).
3. Schranz, D. B. & Lemmark, A. Immunology in diabetes: an update, *Diab*. *Metab*. *Rev*. 14: 3-29 (1998).
4. Tisch, R. & McDevitt, H. Insulin-dependent diabetes mellitus, *Cell* 85: 291-297 (1996).
5. Bach, J. F. Insulin-dependent diabetes mellitus as a β cell targeted disease of immunoregulation, *J*. *Autoimmunity* 8: 439-463 (1995).
6. Rossini, A. A., Greiner, D. L., Friedman, H. P. & Mordes, J. P. Immunopathogenesis of diabetes mellitus, *Diabetes Rev*. 1: 43-75, (1993).
7. The Diabetes Control and Complications Trial Research Group. The effect of intensive treatment of diabetes on the development and progression of long-term complications in insulin-dependent diabetes mellitus, *New Engl. J*. *Med* 329, 977-986 (1993).
8. Cuif, M. H., Doiron, B. & Kahn, A. Insulin and cyclic AMP act at different levels on transcription of the L-type pyruvate kinase gene, *FEBS Lett*. 417, 81-84 (1997).
9. Chen, R., Doiron, B. & Kahn, A. Glucose responsiveness of a reporter gene transduced into hepatocytic cells using a retroviral vector, *FEBS Lett*. 365, 223-226 (1995).
10. Decaux, J. F., Antoine, B. & Kahn, A. Regulation of the expression of the L-type pyruvate kinase gene in adult rat hepatocytes in primary culture, *J*. *Biol*. *Chem*. 264, 11584-11590 (1989).
11. Cuif, M. H., Porteu, A., Kahn, A. & Vaulont, S. Exploration of a liver-specific, glucose/insulin-responsive promoter in transgenic mice, *J*. *Biol*. *Chem*. 268, 13769-13772 (1993).
12. Bergot, M. O., Diaz-Guerra, M. J., Puzenat, N., Raymondjean, M. & Kahn, A. Cis-regulation of the L-type pyruvate kinase gene promoter by glucose, insulin and cyclic AMP, *Nucleic Acids Res.* 20, 1871-1877 (1992).
13. Muzyczka, N. Use of adeno-associated virus as a general transduction vector for mammalian cells, *Curr*. *Top*. *Microbiol*. *Immunol*. 158, 97-129 (1992).
14. Clark, K. R., Liu, X., McGrath, J. P. & Johnson, P. R. Highly purified recombinant adeno-associated virus vectors are biologically active and free of detectable helper and wild-type viruses, *Human Gene Therapy* 10, 1031-1039 (1999).
15. Samulski, R. J. Adeno-associated virus: integration at a specific chromosomal locus, *Curr*. *Opin. Genet*. *Dev.* 3, 74-80 (1993).
16. Giraud, C., Winocour, E. & Berns, K. I. Site-specific integration by adeno-associated virus is directed by a cellular DNA sequence, *Proc*. *Natl*. *Acad*. *Sci. USA* 91, 10039-10043 (1994).
17. Kotin, R. M., Linden, R. M. & Berns, K. I. Characterization of a preferred site on human chromosome 19q for integration of adeno-associated virus DNA by non-homologous recombination, *EMBO J*. 11, 5071-5078 (1992).
18. Studier, F. W., Rosenberg, A. H., Dunn, J. J. & Dubendorff, J. W. Use of T7 RNA polymerase to direct expression of cloned genes, *Methods Enzymol*. 185, 60-89 (1990).
19. Pollet, R. J., Standaert, M. L. & Haase, B. A. Insulin binding to the human lymphocyte receptor. Evaluation of the negative cooperativity model, *J*. *Biol*. *Chem*. 252, 5828-5834 (1977).
20. Roth, J. Assay of peptide hormones using cell receptors: application to insulin and to human growth hormone, *Methods Enzymol*. 37, 66-82 (1975).
21. Frost, S. C. & Lane, M. D. Evidence for the involvement of vicinal sulfhydryl groups in insulin-activated hexose transport by 3T3-L1 adipocytes, *J*. *Biol. Chem.* 260, 2646-2652 (1985).
22. Heath, W. F., *et al*. (A-C-B) human proinsulin, a novel insulin agonist and intermediate in the synthesis ofbiosynthetic human insulin, *J*. *Biol. Chem*. 267, 419-425 (1992).
23. Ma, Z., *et al*. Effect of hemoglobin- and Perflubron-based oxygen carriers on common clinical laboratory tests, *Clin*. *Chem*. 43, 1732-1737 (1997).
24. Yoon, J. W., Rodrigues, M. M., Currier, C. & Notkins, A. Long-term complications of virus-induced diabetes mellitus in mice, *Nature* 296, 566-569 (1982).
25. Yoon, J. W. *et al*. Control of autoimmune diabetes in NOD mice by GAD expression or suppression in cells, *Science* 284, 1183-1187 (1999).
26. Hirasawa K. *et al*. Possible role of macrophage-derived soluble mediators in the pathogenesis of EMC virus-induced diabetes in mice, *J Virol*. 71,4024-4031 (1997).
27. Cameron, N. E., Cotter, M.A. & Low, P.A., Nerve blood flow in early experimental diabetes in rats; realtion to conduction deficits, *Am J*. *Physiol.* 261, E1-E8 (1991).
28. Yoon, J. W., Lesniak, M.A., Fussganger, R. & Notkins, A.L., Genetic differences in susceptibitiy of pancreatic β-cells to virus-induced diabetews mellitus., *Nature* 264, 178-180, (1976).

**Table 1**

| **Functional properties of the recombinant single-chain insulin** | | | | |
|---|---|---|---|---|
| | Insulin receptor binding^{*1} (ED₅₀ in nM) | Glucose uptake^{*2} (ED₅₀ in nM) | *In vivo* hypoglycaemic effect^{*3} (ED₅₀ in nmol/kg) | |
| | | | 1 hour | 2 hours |
| Human insulin | 0.742 (100%)^{§} | 0.322 (100%) | 1.5±0.34 (100%) | 1.8±0.37 (100%) |
| Human proinsulin | 36.1 (2%) | 26.5 (1.25%) | 9.1±1.12 (16.5%) | 9.6±1.28 (18.8%) |
| Single-chain insulin analogue(SIA) | 2.68 (27.7%) | 1.56 (21.3%) | 3.6±0.42 (41.6%) | 3.4±0.18 (52.9%) |

| | | | | |
|---|---|---|---|---|
| *1 ED₅₀ values are presented as the concentration of unlabelled insulin or analogues required to decrease tracer insulin binding to 50% of maximal. | | | | |
| *2 ED₅₀ values are presented as the concentration of insulin or analogues resulting in half-maximal glucose uptake rate. | | | | |
| *3 ED₅₀ values are presented as the dose of insulin or analogues that gave half of the maximal hypoglycaemic activity 1 or 2h after subcutaneous administration to fasted rats. | | | | |
| § The percentage of proinsul in and SIA were determined on the basis of the ED₅₀ value of human insulin as 100%. | | | | |

## Claims

1. A single-chain insulin analog compound of formula (I) having the properties of greater insulin receptor binding activity than proinsulin and less insulin receptor binding activity than insulin:
B chain - Uₗ-Zₙ-Y-Zₗ-Uₙ - A chain (I)
wherein:
B and A chains are the human insulin chains, respectively; and
U is an arginine or lysine residue;
Z is glycine;
l is an integer of 2-n;
n is an integer of 0 or 2; and
Y is glycine-proline-glycine, or
alanine-proline-glycine-aspartic acid-valine, or
tyrosine-proline-glycine-aspartic acid-valine, or
histidine-proline-glycine-aspartic acid-valine.

2. A polynucleotide encoding the single-chain insulin analog according to claim 1.

3. A recombinant vector comprising the polynucleotide according to claim 2.

4. The vector according to claim 3, wherein said vector is a plasmid.

5. The vector according to claim 3, wherein said vector is a virus.

6. The vector according to claim 5, wherein said virus is adeno-associated virus.

7. The vector according to claim 3, comprising an inducible promoter.

8. The vector according to claim 7, wherein said promoter is regulated by glucose.

9. The vector according to claim 8, wherein said promoter is a pyruvate kinase gene promoter.

10. The vector according to claim 9, wherein said promoter is a hepatocyte-specific L-type pyruvate kinase gene promoter.

11. A cell line transformed with the vector according to claim 3.

12. Use of a a recombinant viral or plasmid vector of claim 3-5 comprising a polynucleotide encoding a single-chain insulin analog operatively linked to a promoter; for the manufacture of a medicament for use in remission of diabetes.

13. The use according to claim 12, wherein said viral vector is adeno-associated virus.

14. The use according to claim 13, comprising an inducible promoter.

15. The use according to claim 14, wherein said promoter is regulated by glucose.

16. The method according to claim 13, wherein the dosage of said viral vector is at least about 10¹¹ viral particles.

17. Use of the compound of claim 1 for the manufacture of a medicament for the treatment of diabetes.

18. The use according to claim 17, wherein said diabetes is type I diabetes.

19. The use according to claim 12, wherein said vector is intended for introducing to the patient through the cell line according to claim 11.

## Patentansprüche

1. Einkettige Insulin-Analogverbindung nach Formel (I) mit den Eigenschaften einer höheren Insulinrezeptor-Bindungsaktivität als Proinsulin und einer geringeren Insulinrezeptor-Bindungsaktivität als Insulin:
B-Kette - Uₗ-Zₙ-Y-Zₗ-Uₙ-A-Kette (I)
wobei die B- und A-Ketten jeweils die humanen Insulinketten sind; und
U ein Arginin oder Lysinrest ist;
Z Glycin ist;
l eine ganze Zahl von 2-n ist;
n eine ganze Zahl von 0 oder 2 ist; und
Y Glycin-Prolin-Glycin oder
Alanin-Prolin-Glycin-Asparaginsäure-Valin oder
Tyrosin-Prolin-Glycin-Asparaginsäure-Valin oder
Hystidin-Prolin-Glycin-Asparaginsäure-Valin,
ist.

2. Polynukleotid, das ein einkettiges Insulin-Analog nach Anspruch 1 kodiert.

3. Rekombinanter Vektor, der das Polynukleotid nach Anspruch 2 umfasst.

4. Vektor nach Anspruch 3, wobei der Vektor ein Plasmid ist.

5. Vektor nach Anspruch 3, wobei der Vektor ein Virus ist.

6. Vektor nach Anspruch 5, wobei der Virus ein adenoassoziiertes Virus ist.

7. Vektor nach Anspruch 3, der einen induzierbaren Promoter umfasst.

8. Vektor nach Anspruch 7, wobei der Vektor durch Glucose reguliert wird.

9. Vektor nach Anspruch 8, wobei der Promoter ein Pyruvatkinasegenpromoter ist.

10. Vektor nach Anspruch 9, wobei der Promoter ein Hepatozyten-spezifischer L-Typ Pyruvatkinasegenpromoter ist.

11. Zelllinie, die mit dem Vektor nach Anspruch 3 transformiert worden ist.

12. Verwendung eines rekombinanten Virus- oder Plasmidvektors nach Anspruch 3 bis 5, der ein Polynukleotid umfasst, das ein einkettiges Insulin-Analog funktionell gebunden an einen Promoter kodiert, zur Herstellung eines Medikamentes zur Verwendung in der Remission der Diabetes.

13. Verwendung nach Anspruch 12, wobei der Virus-Vektor ein adenoassoziiertes Virus ist.

14. Verwendung nach Anspruch 13, die einen induzierbaren Promotor umfasst.

15. Verwendung nach Anspruch 14, wobei der Promotor durch Glucose reguliert wird.

16. Verfahren nach Anspruch 13, wobei die Dosierung des viralen Vektors zumindest ungefähr 10¹¹ virale Partikel beträgt.

17. Verwendung der Verbindung nach Anspruch 1 zur Herstellung eines Medikamentes zur Behandlung der Diabetes.

18. Verwendung nach Anspruch 7, wobei die Diabetes Typ I Diabetes ist.

19. Verwendung nach Anspruch 12, wobei der Vektor zur Einbringung in den Patienten durch die Zelllinie nach Anspruch 11 vorgesehen ist.

## Revendications

1. Un composé analogue de l'insuline à chaîne simple de formule (I) ayant comme propriétés une plus grande activité de liaison au récepteur de l'insuline que la pro-insuline et une plus faible activité de liaison au récepteur de l'insuline que l'insuline :
chaîne B - Uₗ - Zₙ - Y - Zₗ - Uₙ - chaîne A (I)
dans laquelle :
les chaînes B et A sont les chaînes de l'insuline humaine, respectivement ; et
U est un résidu d'arginine ou de lysine ;
Z est la glycine ;
l est un nombre entier égal à 2 - n ;
n est un nombre entier égal à 0 ou 2 ; et
Y est glucine - proline - glycine, ou
alanine - proline - glycine - acide aspartique - valine, ou
tyrosine - proline - glycine - acide aspartique - valine, ou
histidine - proline - glycine - acide aspartique - valine.

2. Un polynucléotide codant pour l'analogue de l'insuline à chaîne simple selon la revendication 1.

3. Un vecteur recombinant comprenant le polynucléotide selon la revendication 2.

4. Le vecteur selon la revendication 3, dans lequel ledit vecteur est un plasmide.

5. Le vecteur selon la revendication 3, dans lequel ledit vecteur est un virus.

6. Le vecteur selon la revendication 5, dans lequel ledit vecteur est un virus adéno-associé.

7. Le vecteur selon la revendication 3, comprenant un promoteur inductible.

8. Le vecteur selon la revendication 7, dans lequel ledit promoteur est régulé par le glucose.

9. Le vecteur selon la revendication 8, dans lequel ledit promoteur est un promoteur de gène pyruvate kinase.

10. Le vecteur selon la revendication 9, dans lequel ledit promoteur est un promoteur de gène pyruvate kinase de type L spécifique de l'hépatocyte.

11. Une lignée cellulaire transformée au moyen du vecteur selon la revendication 3.

12. Utilisation d' un vecteur recombinant viral ou plasmide des revendications 3 - 5 comprenant un polynucléotide codant pour un analogue de l' insuline à chaîne simple lié de façon opérationnelle à un promoteur pour la fabrication d' un médicament destiné à l'utilisation pour la rémission du diabète.

13. Utilisation selon la revendication 12, dans laquelle ledit vecteur viral est un virus adéno-associé.

14. Utilisation selon la revendication 13, comprenant un promoteur inductible.

15. Utilisation selon la revendication 14, dans laquelle ledit promoteur est régulé par le glucose.

16. Utilisation selon la revendication 13, dans laquelle le dosage dudit vecteur viral est d'au moins environ 10¹¹ particules virales.

17. Utilisation du composé de la revendication 1 pour la fabrication d'un médicament pour le traitement du diabète.

18. L' utilisation selon la revendication 17, dans laquelle ledit diabète est un diabète de type I.

19. L' utilisation selon la revendication 12, dans laquelle ledit vecteur est destiné à être introduit dans le patient à travers la lignée cellulaire selon la revendication 11.
